# EUROPÄISCHE PATENTSCHRIFT

(11) **EP 0 945 136 B1**
(45) Veröffentlichungstag und Bekanntmachung des Hinweises auf die Patenterteilung: **16.11.2005**
(21) Anmeldenummer: 99104009.8
(22) Anmeldetag: 12.03.1999
(51) Int. Cl.: A61K 38/13, A61K 9/107, A61P 17/00, A61P 37/08

(54) **Topisches Arzneimittel mit einem Gehalt an Ciclosporin**
Topical pharmaceutical preparation comprising ciclosporin
Composition pharmaceutique topique comprenant de la ciclosporine

(30) Priorität: 12.03.1998 DE 19810655
(43) Veröffentlichungstag der Anmeldung: 29.09.1999
(73) Patentinhaber: Heide, Peter Edgar, Dr., 72147 Nehren (DE)
(72) Erfinder: Heide, Peter Edgar, Dr., 72147 Nehren (DE)
(74) Vertreter: Möbus, Daniela

(56) Entgegenhaltungen:
- EP-A- 0 327 280
- EP-A- 0 651 995
- WO-A-95/31969
- WO-A-97/25977
- DE-A- 19 537 012
- US-A- 5 342 625
- US-A- 5 660 858
- PATENT ABSTRACTS OF JAPAN Bd. 011, Nr. 101 (C-413) 31 M{rz 1987 & JP 61 249 918 A (YUTAKA MIZUSHIMA) 07 November 1986
- DATABASE WPI Section Ch, Week 198651, Derwent Publications Ltd., London, GB; Class B05, AN 1986-335072 'Eye drops - composed of lipid microspheres containing remedies for eye troubles' & JP 61 249 918 A (MIZUSHIMA H.) 07 November 1986
- DATABASE BIOSIS [Online] BIOSIS INFORMATION SERVICE, PHILADELPHIA, PA, US; NEUMANN RON ET AL: 'Immunohistopathologic features and therapy of conjunctival lichen planus' PREV199396020750 *a AMERICAN JOURNAL OF OPHTHALMOLOGY (1993), 115(4), 494-500
- STÜTTGEN G.: 'Ciclosporin bei therapierefraktärer Neurodermitis atopica' DEUTSCHE MEDIZINISCHE WOCHENSCHRIFT Bd. 117, Nr. 20, 15 Mai 1992, DEUTSCHLAND, Seiten 804 - 805, XP001146576
- HINGORANI M. ET AL: 'Therapeutic options in ocular allergic disease' DRUGS Bd. 50, Nr. 2, 1995, ADIS INTERNATIONAL LTD, AT, Seiten 208 - 221, XP002112535

## Beschreibung

Die Erfindung betrifft die Verwendung von Ciclosporin in einer zur topischen Anwendung geeigneten Galenik zur Herstellung eines Arzneimittels zur Behandlung von Allergien, zur Behandlung von Hantkrankheiten, zur Behandlung von Krankheiten der Mundschleimhaut oder zur Behandlung der Schleinhante des Genitalbereichs, wobei das Ciclosporin in einer Öl-in-Wasser-Nanoemulsion vorliegt und die Nanoemulsion einen Gehalt an Triacylglyceriden, vorzugsweise mittelkettigen Triacylglyceriden, aufweist, wobei als Emulgator lediglich Lecithin vorhanden ist und wobei es einen Gehalt an zumindest einem viskositätserhöhenden Zusatz aufweist. Arzneimittel mit einem Gehalt an Ciclosporin sind allgemein zur Behandlung von Transplantationspatienten bekannt.

Der Wirkstoff Ciclosporin ist ein zyklisches, aus elf Aminosäuren bestehendes Peptid, mit der Summenformel C₆₂H₁₁₁N₁₁O₁₂. Es wird auch als Ciclosporin A (WHO) bezeichnet. Ursprünglich wurde es aus Pilzen isoliert. Inzwischen sind auch Verfahren zu einer synthetischen Herstellung bekannt.

Ciclosporin ist ein so genannter Immunmodulator mit immunsuppressiver Wirkung. Es blockiert die Aktivierung von Helfer- und Killerzellen des Immunsystems durch Inhibition der Lymphokinproduktion. Ciclosporin unterdrückt dabei sowohl die humorale als auch die zelluläre Immunreaktion, indem es die Freisetzung von Interleukinen, insbesondere von IL-1 aus Monozyten und IL-2 aus T-Helfer-Zellen in den frühen Phasen der Immunantwort unterbindet.

Aufgrund dieser immunsuppressiven Wirkung wird Ciclosporin zur Vorbeugung von Transplantatabstoßung nach allogenen Transplantationen von Niere, Leber, Herz, Herz-Lunge, Lunge und Pankreas sowie nach Knochenmark-Transplantationen eingesetzt.

Außerdem wird Ciclosporin zur Behandlung der Graft-Versus-Host-Krankheit eingesetzt, einer Krankheit, die bei Transplantationspatienten nach Übertragung fremder immunkompetenter Zellen durch zelluläre Immunreaktionen auftritt.

Weitere Anwendungsgebiete für Ciclosporin sind die Behandlung von schwerer endogener Uveitis, einer schweren Entzündung der Aderhaut des Auges, sowie von schwersten therapieresistenten Formen der Psoriasis (Schuppenflechte). Auch die therapeutische Wirksamkeit von Ciclosporin zur Behandlung des steroidabhängigen und steroidresisteten nephrotischen Syndroms, also von Nierenerkrankungen, die mit einem ausgeprägten Eiweißverlust einhergehen, sind bekannt.

Ciclosporin wurde als Infusionslösung sowie als Trinklösung von der Firma Sandoz bzw. Novartis AG, Basel, Schweiz, dargereicht. Da Ciclosporin ein hydrophobes Peptid ist, das in wässriger Lösung nicht lösbar ist, enthielten die bisherigen Darreichungsformen als Emulgatoren bzw. Löslichkeitsvermittler in großen Mengen Ethanol (ca. 12 Vol.-%) sowie Lipide in Form von Maiskeimöl und Triacylglycerid-Derivaten.

Diese bekannten Verabreichungsformen von Ciclosporin sind lediglich zur systemischen Anwendung geeignet. Wenn Ciclosporin in Form einer Trinklösung verabreicht wird, so erfolgt die Aufnahme in den Körper über den Darm. Bei einer Infusion der Ciclosporin-Lösung gelangt der Wirkstoff direkt ins Blut und verteilt sich über das Blut im gesamten Körper.

Eine topische, d. h. örtlich begrenzte Anwendung von Ciclosporin ist aufgrund seiner lipophilen Eigenschaften, die die Verwendung von Ethanol und Lipiden zum Löslichmachen des Ciclosporins erfordern, problematisch.

Eine pharmazeutische Zubereitung, die unter anderem auch für Ciclosporin vorgeschlagen wird, ist in der US-A-5,154,930 beschrieben. Diese Verabreichungsform umfasst ein salzfreies geladenes Lipid, wie beispielsweise Phosphatidylethanolamin oder Phosphatidylserin sowie ein Lösungsmittel wie Polyethylenglykol oder Ethanol. Dabei bilden sich in der pharmazeutischen Zusammensetzung Liposomen-Komplexe zwischen dem Wirkstoff und den Lösungsmitteln. Die beschriebene pharmazeutische Zubereitung erlaubt es, besonders hohe Konzentrationen an Wirkstoff, beispielsweise Ciclosporin, zu verabreichen.

Als Verabreichungsformen werden Tabletten, Kapseln, Dragees und Ähnliches vorgeschlagen. Zur örtlich begrenzten Verwendung an besonders empfindlichen Körperbereichen ist diese Darreichungsform aufgrund der Anwesenheit des zum Löslichmachen der hydrophoben Wirkstoffe notwendigen Lösungsmittels Ethanol bzw. Polyethylenglykol jedoch nicht geeignet.

Aus der US-A-5,472,706 ist eine gefriergetrocknete Zusammensetzung bekannt, die dadurch erhalten wurde, dass eine Öl-in-Wasser-Emulsion gefriergetrocknet wird, die Wasser, 0,2 bis 25 Gew.-% eines Öles, 0,1 bis 10 Gew.-% eines Emulgators und 0,25 bis 50 Gew.-% eines Kälteschutzmittels enthält, das Aminosäuren enthält und die in einer Menge von zumindest der Menge des Öles vorhanden sind.

Diese trockene Mischung dient dazu, um später flüssige Makro- bzw. Mikroemulsionen daraus herzustellen, die Ciclosporin enthalten können.

Ein Arzneimittel, das aus dieser gefriergetrockneten Substanz hergestellt wird, enthält zwangsläufig das Kälteschutzmittel, welches pharmakologisch bedenklich und damit unerwünscht ist.

Aus der US-A-5,342,625 ist eine pharmazeutische Zusammensetzung bekannt, die Ciclosporin enthält und die in Form eines Mikroemulsionsvorkonzentrates bzw. in der Form einer Mikroemulsion vorliegt und die in der hydrophilen Phase C₁₋₅-Alkyl- oder Tetrahydrofurfuryl-di- oder -partial-Ether von Mono- oder Polyloxyalkandiolen niederen Molekulargewichts, nämlich Transcutol oder Glykofurol enthält.

Diese Ether sind pharmakologisch bedenklich und damit unerwünscht.

Mikroemulsionen im strengen Sinne sind thermodynamisch stabile Mizellmischungen. Mikroemulsionen benötigen zur Herstellung üblicherweise zwei Tenside, nämlich ein Tensid und ein Co-Tensid, um die Besonderheit dieses physikalisch-chemischen Phänomens zu erreichen. Der Begriff "Mikroemulsion" bezieht sich auf ein Mehrkomponentensystem aus Wasser/Öl/Tensid/Co-Tensid, das sich spontan bildet und als optisch isotropes, transparentes, flüssiges Mehrstoffsystem in Erscheinung tritt. Es handelt sich dabei um ein Vierkomponentensystem, das thermodynamisch stabil, transparent und isotrop ist und ein reversibles Temperaturverhalten zeigt, wobei das Fließverhalten von Mikroemulsionen dem Newton'schen Gesetz folgt, und diese weisen extrem niedrige, positive Grenzflächenspannungswerte auf.

Im Gegensatz dazu sind Nanoemulsionen thermodynamisch instabil, dafür kinetisch stabil, es handelt sich um Dreikomponentensysteme, bei denen eine innere disperse Phase feinverteilt in einer äußeren, mit der inneren nicht mischbaren Phase vorliegt. Das Zusammenfließen der Innenphase zu einer größeren wird durch den Einsatz von Emulgatoren stark verzögert.

Bei den aus WO 95/31969 A bekannten Präparaten handelt es sich um pharmazeutische von Gelen auf Mikroemulsionsbasis abgeleitete pharmazeutische Präparate. Weiterhin ist ein Verfahren für deren Herstellung beschrieben. Die bekannten Mikroemulsionen werden durch Zugabe von Ethanol in ihren hydrophilen Eigenschaften eingeschränkt und in einem System wie diesem liefert das Lecithin stabile Mikroemulsionen. Der zugegebene Alkohol wirkt als amphiles Lösungsmittel.

Aus der EP-A-0 696 452 ist ein Arzneimittel bekannt, das in Form einer Nanoemulsion vorliegt und das einen Gehalt an Ciclosporin aufweist. Die darin eingesetzten Emulgatoren sind nichtionogene Tenside der Polyethylenglykol-Blockpolymer-Klasse, die völlig körperfremd sind. Als weitere Beispiele für Emulgatoren sind Komplexbildner erwähnt, die sich in der menschlichen Zelle anreichern können, insbesondere das darin beschriebene Nutrium-EDTA, wobei in der Zelle durch die komplexierende Eigenschaft eine Inaktivierung metallbeladener Enzyme, eine Komplexierung von physiologisch wertvollen Erdalkali- und Metallionen erfolgt und ferner eine hohe Verweildauer dieser Komplexe im menschlichen Körper vorhanden ist. Die eingesetzten Emulgatoren sind somit körperfremde Stoffe, die pharmakologisch unerwünscht sind.

Der WPI- und PAY - Abstrakt der Patentschrift JP 61249918 A beschreibt Augentropfen mit Cyclosporin in einer Nanoemulsion, wobei alsLipid Sojabohnenöl eingesetzt wird.

Die US-A-5 660 858 beschreibt ebenfalls ein Arzneimittel mit Ciclosporin in einer Nanoemulsion mit einem Gehalt an Triacylglyceriden und mit Eilecithin sowie weiteren Phospholipiden als Emulgatoren. Dieses Arzneimittel ist vor allem für Injektionen gedacht und weist keine Trägerstoffe zum lokalen Auftragen auf. Insbesondere fehlt es an viskosizitätserhöhenden Zusätzen, die einen Verbleib des Ciclosporin am gewünschten Wirkort verlängern.

Die topische Verwendung von hydrophoben Wirkstoffen ist immer dort problematisch, wo stark wasserhaltige bzw. hydrophile Körperteile behandelt werden sollen, da eine Voraussetzung zur Aufnahme solcher Wirkstoffe in den Körper darin besteht, zunächst einen Kontakt zwischen Wirkstoff und Körperoberfläche herzustellen. Da Emulsionen nur in gewissen Konzentrationsbereichen der Bestandteile stabil sind, besteht die Gefahr, dass wasserhaltiges Gewebe die Emulsion spalten kann.

Vor diesem Hintergrund ist es die Aufgabe der Erfindung, ein Arzneimittel mit einer topischen Zusammensetzung, einen hydrophoben Wirkstoff, nämlich Ciclosporin, enthaltend, bereitzustellen, welches an stark wasserhaltigen und/oder sehr empfindlichen Körperbereichen verabreicht werden kann, eine gute Wirkstoffaufnahme ermöglicht und auf physiologisch bedenkliche bzw. unerträgliche Hilfs-, Begleit- oder Lösungsmittel verzichtet.

Erfindungsgemäß wird die Aufgabe mit einem Arzneimittel mit den Merkmalen der Ansprüche 1 bis 13 gelöst.

Wirkstoff Ciclosporin optimal verteilt. Bei einer Applikation in stark wasserhaltigen Körperbereichen kann so eine besonders gute Verteilung von Ciclosporin und damit eine optimale Wirkstoffaufnahme erreicht werden.

So zeigte sich in einer über sechs Monate andauernden Studie in der Universitätsklinik Tübingen, dass die Verwendung einer erfindungsgemäßen Nanoemulsion sowohl im Bereich des hochempfindlichen Auges als auch im Hautbereich eine gegenüber herkömmlichen Therapieformen wesentlich verbesserte Wirksamkeit und Verträglichkeit aufweist. Dies ist vor allem darauf zurückzuführen, dass aufgrund der neuen Darreichungsform auf die Verwendung physiologisch bedenklicher bzw. unverträglicher Hilfs-, Begleit- oder Lösungsmittel vollständig verzichtet werden kann, ohne dass dadurch die Wirkung oder Aufnahme von Ciclosporin an den betreffenden Körperteilen beeinträchtigt wird.

Durch die Verwendung von Leicthin als Phospholipid in dem erfindungsgemäßen Arzneimittel weist dieses eine besonders hohe physiologische Verträglichkeit auf. Dadurch ist das Arzneimittel auch an besonders sensiblen Organen wie beispielsweise dem menschlichen Auge einsetzbar.

Lecithin oder Phosphatidylcholin ist eines der am weitesten verbreiteten Membranlipide des Menschen. Von der WHO ist dem Lecithin Unbedenklichkeit als Lebensmittel zuerkannt worden, es wurden keine ADI-Werte (Acceptable Daily Intake) zuerkannt. Lecithin entspricht ferner den Normen der US-amerikanischen Behörde FDA und besitzt den GRAS-Status (Generally Recognized As Safe, CFR Nr. 182.1400/184.1400).

In Fettemulsionen für die parenterale Ernährung wird Lecithin in Kliniken in großem Umfang eingesetzt.

Unter einer Nanoemulsion im Sinne der Erfindung wird jede Öl-in-Wasser-Emulsion verstanden, die Tröpfchengrößen im Nanometerbereich, also mit Durchmessern von kleiner als 1 µm enthält. Derartige Nanoemulsionen haben eine ölige bzw. Lipid-Phase und eine wässrige Phase, wobei die wässrige Phase Wasser oder physiologisch verträgliche wässrige Lösungen wie beispielsweise physiologische Kochsalzlösung (0,9 Gew.-% Natriumchlorid in Wasser) aufweist.

In einer derartigen Nanoemulsion wird der hydrophobe Wirkstoff Ciclosporin in den winzigen öligen Tröpfchen gelöst, die wiederum in der wässrigen Phase dispergiert sind. Somit ist der Lecithin ist somit ein besonders gut verträglicher Emulgator, der aufgrund seines Vorkommens in menschlichen Zellen ohnehin Bestandteil des menschlichen Körpers und damit gesundheitlich unbedenklich ist. Als Emulgator von Ciclosporin ist es aufgrund seiner stark amphiphilen Eigenschaften besonders gut geeignet.

In einer weiteren Ausgestaltung der Erfindung liegt der Gehalt an Lecithin im Bereich von 0,1 bis 20 Gew.-%, vorzugsweise im Bereich von 1 bis 10 Gew.-%.

Hierbei ist vorteilhaft, dass diese Konzentrationen eine besonders feine Emulgierung des Ciclosporins in einer wässrigen Lösung erlauben.

Die Nanoemulsion weist außerdem einen Gehalt an Triacylglyceriden, bevorzugt mittelkettigen Triacylglyceriden auf.

Triacylglyceride sind neutrale Lipide, bei denen Fettsäuren über Esterbindungen an einen Glycerinrest gebunden sind. Die Fettsäuren können kurz-, mittel- oder langkettig sein, sie können gesättigt oder ungesättigt vorliegen. Triacylglyceride sind stark hyrophobe Stoffe und dienen z. B. als Energiespeicher im Körper, wo sie in den Fettzellen abgelagert werden.

Die Verwendung von Triacylglyceriden, insbesondere mittelkettigen Triacylglyceriden hat den Vorteil, dass diese problemlos in Arzneimittelqualität erhältich und zum Inlösungbringen des Ciclosporin in einer Nanoemulsion besonders gut geeignet sind.

Dies gilt vor allem dann, wenn der Gehalt an Triacylglyceriden im Bereich von 10 bis 40 Gew.-%, bevorzugt im Bereich von 20 bis 30 Gew.-% vorliegt.

In einer besonders vorteilhaften Ausgestaltung liegt der Gesamtgehalt an Lipiden im Bereich von 1 bis 50 Gew.-%, bevorzugt im Bereich von 20 bis 30 Gew.-%.

Dabei umfasst der Gesamtgehalt an Lipiden sowohl rein hydrophobe Lipide wie Triacylglyceride als auch Lecithin.

Diese Maßnahme hat den Vorteil, dass bei einem möglichst effizienten Emulgieren des hydrophoben Wirkstoffs Ciclosporin gleichzeitig eine Anwendung an hydrophilen Oberflächen und eine gute Wirkstoffaufnahme möglich ist.

In einer weitern Ausgestaltung der Erfindung liegt der Gehalt an Ciclosporin im Bereich von 0,1 bis 10 Gew.-%, bevorzugt im Bereich von 1 bis 3 Gew.-%.

Hierbei ist vorteilhaft, dass eine gute therapeutische Wirksamkeit von Ciclosporin erzielt wird.

In einer besonders vorteilhaften Ausgestaltung weist das Arzneimittel 2 Gew.-% Ciclosporin, 5 Gew.-% Lecithin, 23 Gew.-% mittelkettige Triacylglyceride und 70 Gew.-% physiologische Kochsalzlösung sowie ggf. ein physiologisch verträgliches Konservierungsmittel auf.

Es hat sich nämlich in einer klinischen Studie mit dem erfindungsgemäßen Arzneimittel herausgestellt, dass mit dieser Zusammensetzung eine besonders gute Verträglichkeit bei optimaler Wirkstoffaufnahme in den Körper erreicht wird.

Der Zusatz eines Konservierungsmittels ist notwendig, wenn das Arzneimittel für längere Zeitspannen aufbewahrt werden soll. Es versteht sich, dass Konservierungsmittel bei allen Verabreichungsformen des erfindungsgemäßen Arzneimittels enthalten sein können.

In einer weiteren Ausgestaltung liegt das erfindungsgemäße Arzneimittel mit physiologisch verträglichen Trägerstoffen zum Auftragen auf die Haut vermischt vor.

Derartige Trägerstoffe können beispielsweise Cremes, Gele oder Salben mit den üblichen Bestandteilen sein.

Hierbei ist von Vorteil, dass bei der Verwendung der erfindungsgemäßen Nanoemulsion auf der Haut oder einer Schleimhaut die Verteilung sowie das Zurückhalten auf der Haut verbessert wird. Darüber hinaus wird die Anwendung des Arzneimittels für den Patienten erleichtert.

Das Arzneimittel weist außerdem viskositätserhöhende Zusätze auf.

Derartige Zusätze können beispielsweise Zellulosederivate, Polyacrylate oder andere physiologisch verträgliche Polymere sein.

Hierbei ist von Vorteil, daß der Verbleib des Wirkstoffs Ciclosporin an dem Ort, wo er wirken soll, verlängert wird.

Die Erfindung betrifft auch die Verwendung von Ciclosporin in einer zur topischen Anwendung geeigneten Galenik zur Herstellung eines Arzneimittels gemäß Anspruch 1 zur Behandlung von Hautkrankheiten.

Bei in der Hautklinik der Universitätsklinik Tübingen durchgeführten Versuchen wurde nämlich erstmals die topische Anwendung von Ciclosporin zur Behandlung von Hautkrankeiten untersucht. Dabei stellte sich heraus, daß durch die topische Verabreichung von Ciclosporin gegenüber den üblicherweise verwendeten Therapien, insbesondere der Verabreichung von Cortisonpräparaten, eine überlegene therapeutische Wirkung bei gleichzeitiger guter Verträglichkeit erzielt wird.

Da Ciclosporin bisher nur systemisch angewendet wurde, kam die Behandlung von gewöhnlichen lokal begrenzten Hautkrankheiten aufgrund der schweren Nebenwirkungen einer systemischen Anwendung nicht in Betracht. Bei der systemischen Verabreichung kommt es aufgrund der immunsupprimierenden Wirkung des Ciclosporins nämlich zu einer erhöhten Anfälligkeit gegen Infektionen jeglicher Art. Diese Nebenwirkungen wurden bisher nur bei schweren, sonst nicht behandelbaren Krankheiten in Kauf genommen.

Galeniken, die bei einer topischen Verwendung in Betracht kommen, umfassen z.B. die Formulierung als Cremes, Gele, Salben oder auch in Form von Liposomen oder Mikroemulsionen.

Erfindungsgemäß ist jedoch die Verwendung von Ciclosporin in Form einer Nanoemulsion, wie sie weiter oben beschrieben wurde.

Hierbei ist vorteilhaft, dass sich der Wirkstoff Ciclosporin bei einer Verabreichung als Nanoemulsion in der oberen Hautschicht, der Hornschicht, anreichert. Dadurch wird ein besonders langer Wirkstoffverbleib in diesen Hautbezirken erreicht, was erwünscht ist, da bei den meisten Hautkrankheiten die obersten Hautzellschichten befallen sind.

Die Erfindung betrifft auch die Verwendung von Ciclosporin in einer zur topischen Anwendung geeigneten Galenik zur Herstellung eines Arzneimittels gemäß Anspruch 1 zur Behandlung von Krankheiten der Mundschleimhaut und/oder der Schleimhäute des Genitalbereichs.

Im Bereich dieser stark wasserhaltigen Oberflächen ist eine rasche Aufnahme es hydrophoben Wirkstoffs zwingend erforderlich, da er an diesen Körperoberflächen nicht anhaftet und vor allem im Mundbereich durch Speichel schnell weggespült wird. Die erfindungsgemäße Nanoemulsion sorgt dabei für die Anlagerung von Ciclosporin an die Schleimhäute und fördert somit eine schnelle Aufnahme.

In einer weiteren vorteilhaften Ausgestaltung wird das Arzneimittel zur Behandlung von Lichen ruber eingesetzt.

Diese Krankheit ist eine sehr verbreitete endzündliche Erkrankung der Haut und Schleimhaut, die auch als kleinpapulöses Exanthem oder Flechte bezeichnet wird.

Zur Behandlung dieser Hautkrankheit wurden bisher lediglich Schälkuren mit Vitamin A-Säure und anschließende Hydrocortisonbehandlung oder Behandlung mit anderen Cortisonpräparaten eingesetzt. Im Genitalbereich waren zur Behandlung von Lichen ruber bisher sogar operative Eingriffe erforderlich, die durch die topische Anwendung von Ciclosporin nun unterbleiben können.

In einer weiteren Ausgestaltung wird das erfindungsgemäße Arzneimittel zur Behandlung von Neurodermitis eingesetzt.

Bei einem Vergleich der Ciclosporin-Anwendung und der bisher üblichen Hydrocortisonanwendung konnten verbesserte Therapieerfolge mit Ciclosporin bei der Behandlung von Neurodermitis beobachtet werden.

In einer weiteren vorteilhaften Ausgestaltung wird das Arzneimittel zur Behandlung von Neurodermitis im Bereich des Auges verwendet.

Hierbei ist vorteilhaft, dass Ciclosporin, insbesondere wenn es in Form einer erfindungsgemäßen Nanoemulsion vorliegt, keine Reizungen im Auge oder in den Bereichen um das Auge herum hervorruft, wobei es gleichzeitig gut aufgenommen wird, und dass es hoch effizient gegen Neurodermitis im Augenbereich wirkt, wie in an der Augenklinik der Universitätsklinik Tübingen durchgeführten Versuchen mit über 500 Patienten nachgewiesen werden konnte.

Die Erfindung betrifft auch die Verwerdung von Ciclosporin in einer zur topischen Anwendung geeigneten Galenik zur Herstellung eines Arzneimittels gemäß Anspruch 5 zur Behandlung von Allergien.

In einer an der Augenklinik der Universitätsklinik Tübingen durchgeführten Studie zeigte sich nämlich, daß Ciclosporin bei topischer Anwendung therapeutisch hochwirksam gegen Allergien eingesetzt werden kann. Ciclosporin kann dabei in allen zur topischen Verabreichung geeigneten Galeniken eingesetzt werden. Besonders bevorzugt ist dabei eine Darreichung als Nanoemulsion, wie sie oben näher beschrieben wurde.

Insbesondere bei einer Verabreichung von Ciclosporin als Nanoemulsion zur Bekämpfung von Allergien im Augenbereich konnten hervorragende Therapieerfolge erzielt werden.

In einer weiteren vorteilhaften Ausgestaltung wird das erfindungsgemäße Arzneimittel zur prophylaktischen und/oder therapeutischen Behandlung des Auges verwendet.

Eine therapeutische Behandlung des menschlichen Auges mit Ciclosporin ist z.B. bei Hornhaut-Transplantationen zur Verhinderung von Abstoßungsreaktionen erforderlich.

Bei der Verwendung von Ciclosporin als Nanoemulsion verteilt sich der hydrophobe Wirkstoff besonders gut über den gesamten Augapfel, so daß aufgrund der großen Resorptionsfläche eine optimale Wirkstoffaufnahme gegeben ist. Die Nanoemulsion verteilt sich außerdem im Kammerwasser selbst, das auch die Linse und die Hornhaut umspült. Da das Kammerwasser nur ca. alle vier Stunden ausgetauscht wird, kann Ciclosporin besonders dauerhaft auf die von dem Kammerwasser benetzten Augenbereiche einwirken. So kann das Risiko von Gewebeabstoßungen im Bereich des Auges sicher vermieden werden.

In klinischen Versuchen, bei denen die Verwendung des erfindungsgemäßen Arzneimittels am Auge getestet wurde, kam es in keinem einzigen Fall zu Sehbeeinträchtigungen oder einer Verstopfung der Schlemm-Kanäle, die dem Abfließen der Tränenflüssigkeit in die Nase dienen. Darüber hinaus wurden keine Schmerzfälle beobachtet.

In einer besonders vorteilhaften Ausgestaltung wird das erfindungsgemäße Arzneimittel zur Verhinderung von Abstoßungsreaktionen nach Transplantationen, vorzugsweise im Bereich des Auges, verwendet.

Hierbei ist bspw. an die bereits erwähnten Hornhaut-Transplantationen oder Transplantationen anderer Bestandteile des Auges, jedoch auch an Haut-Transplantationen zu denken.

Da der Wirkstoff nun direkt am Zielort aufgetragen werden kann und dort auch gut aufgenommen wird, kann das Ciclosporin mit im Vergleich zur systemischen Anwendung geringen Nebenwirkungen effizient therapeutisch wirken.

In einem Verfahren zur Zubereitung des erfindungsgemäßen Arzneimittels werden die folgenden grundsätzlichen Schritte durchgeführt:
a) Lösen von Ciclosporin in einer öligen Phase;
b) Hinzufügen eines Anteils einer wäßrigen Phase;
c) Rühren;
d) Hinzufügen des verbleibenden Anteils der wäßrigen Phase;
e) Behandeln des Gemischs mit Ultraschall; und
f) Sterilfiltrieren.

Hierbei ist von Vorteil, daß eine erfindungsgemäße Nanoemulsion mit Ciclosporin in einem zügigen Verfahren ohne technischen Aufwand hergestellt werden kann.

Die ölige Phase kann dabei z.B. Triacylglyceride und Lecithin, die wäßrige Phase physiologische Kochsalzlösung oder Wasser enthalten. Das Lösen des Ciclosporins in Schritt a) sowie das Rühren in Schritt c) kann z.B. durch auf einem Magnetrührer oder mit einem Flügelrührer durchgeführt werden. Es versteht sich, daß das erfindungsgemäße Verfahren unter sterilen Bedingungen durchgeführt werden muß, wobei zwischen den einzelnen aufgeführten Schritten jeweils Sterilfiltrationsschritte zwischengeschaltet werden können.

Die Ultraschallbehandlung dient der Dispersion der öligen Phase in der wäßrigen Phase, wobei die Tröpfchengrößen in der entstehenden Suspension durch die Dauer der Ultraschallbehandlung und die Leistung bestimmt wird.

Weitere Merkmale und Vorteile der Erfindung ergeben sich aus den nachfolgenden Ausführungsbeispielen.

### Beispiel 1 Herstellung einer Ciclosporin-Nanoemulsion

Es wird eine Ciclosporin-Nanoemulsion hergestellt, die aus 2 Gew.-% Ciclosporin, 23 Gew.-% Oleum neutrale DAB, 5 Gew.-% Lecithin und 70 Gew.-% 0,9%iger Natriumchloridlösung besteht.
Die fertige Emulsion ist u.a. zur Verwendung als Ausgentropfen geeignet.

### Inhaltsstoffe und ihre Bezugsquellen:

| | |
|---|---|
| Ciclosporin | Firma Synochem, Hamburg; |
| Oleum neutrale DAB (MIGLYKOL) | Firma Henkel, Düsseldorf; |
| Lecithin (80 Gew.-% Phosphatidylcholin) | Firma Lipoid, Ludwigshafen; |
| Natriumchlorid, 0,9 Gew.-% | Firma Braun, Melsungen. |

Alle verwendeten Hilfsmittel, bspw. Bechergläser, Rührer, Filter, usw. werden bei 121°C für 15 Minuten lang durch Autoklavieren sterilisiert.

Zunächst wird der lipophile Wirkstoff Ciclosporin zusammen mit dem Lecithin in Oleum neutrale gelöst.

Dazu werden in ein steriles Becherglas 5 g Lecithin, 2 g Ciclosporin und 23 ml Oleum neutrale eingefüllt und mit einem Magnetrührer in Lösung gebracht.

Die Lösung wird in ein zweites Becherglas sterilfiltriert. Dann werden 40 ml 0,9%ige Natriumchloridlösung zugefügt und für 1 Stunde bei 400 upm (Umdrehungen pro Minute) mit einem Flügelrührer gerührt.

Dadurch wird eine Voremulsion erzeugt, die in eine sterile Durchflußzelle gegeben und mit der restlichen Menge an 0,9%iger Natriumchloridlösung aufgefüllt wird.

Diese Lösung wird für 15 Minuten mit 70 Watt Leistung in einem Ultraschallgenerator (Firma Branson, Schwäbisch Gmünd) beschallt.

Dabei wird eine Nanoemulsion mit Tröpfchengrößen von kleiner als 500 nm erzeugt, die über einen 0,45 µm-Sterilfilter direkt in Augentropfflaschen abgefüllt wird.

Alle Arbeitsschritte werden unter einer sterilen Werkbank (Laminar Airflow Bank, Firma Ehret, Emmendingen) durchgeführt.

Die Augentropfen sind bei einer Lagertemperatur von 4°C für drei Monate lang steril.

### Beispiel 2 Studie mit Patienten an der Universitätsklinik Tübingen

### 1. Augenklinik

In der Augenklinik der Universitätsklinit Tübingen wurden die in Beispiel 1 hergestellten Augentropfen für einen Zeitraum von sechs Monaten mit insgesamt über 200 Präparationen bei Patienten mit Hornhaut-Transplantationen eingesetzt. Die Studie dauert noch an.

Über den gesamten Zeitraum der Behandlung von Patienten mit dem erfindungsgemäßen Arzneimittel in Form von Augentropfen wurde kein einziger Fall von Schmerzentwicklung bei der Verabreichung der Tropfen beobachtet.

Obwohl die Nanoemulsion ein milchiges Aussehen aufweist, kam es bei der Applikation am Auge in keinem Fall zu Sehbeeinträchtigungen.

Die erfindungsgemäße Ciclosporin-Nanoemulsion wurde außerdem zur Behandlung von Neurodermitiden im Bereich des menschlichen Auges sowie zur Behandlung von Allergien im Augenbereich eingesetzt. Bei beiden Krankheitsbildern konnten überragende Therapieerfolge erreicht werden, ohne daß es zu einer Entwicklung von Schmerzen oder Sehbeeinträchtigungen bei den Patienten gekommen wäre.

### 2. Hautklinik

Die Ciclosporin-Nanoemulsion wurde darüber hinaus vier Monate lang in der Hautklinik der Universitätsklinik Tübingen zur Behandlung von Lichen ruber im Gesichtsbereich und im Genitalbereich eingesetzt.

Durch den Einsatz von Ciclosporin bei der Behandlung im Gesichtsbereich konnten die bisher üblichen Schälkuren mit Vitamin A-Säure und anschließender Hydrocortisonbehandlung vermieden werden.

Bei der Verwendung im Genitalbereich war es darüber hinaus möglich, auf die bisher üblichen operativen Eingriffe zu verzichten.

Die erfindungsgemäße Ciclosporin-Nanoemulsion wurde außerdem zur Behandlung von Neurodermitis eingesetzt. Hier konnte ein verbesserter Therapieerfolg im Vergleich zu einer Hydrocortisonbehandlung erreicht werden.

In der Hautklinik wurde das erfindungsgemäße Arzneimittel sowohl stationär als auch ambulant eingesetzt. Auch die Studien in der Hautklinik dauern noch an. Darüber hinaus werden derzeit Versuche zur Verabreichung von Ciclosporin zur Behandlung von Hautkrankheiten in Form von Liposomen durchgeführt.

Seit einem Jahr läuft eine Neurodermitis-Studie an der Universität-Hautklinik in Tübingen. Die Arzneimittel wurden großflächig jeweils über drei Wochen am erkrankten Patienten eingesetzt. Bisher konnte in keinem einzigen Fall Ciclosporin im Blut der Patienten nachgewiesen werden (Erfassungsgröße > 20 ng/ml). Dies bedeutet, das Arzneimittel bleibt am zu behandelnden Gewebe, wirkt nicht systemisch und kann daher bei optimaler Wirksamkeit mit möglichst wenig Wirkstoff auskommen.

## Patentansprüche

1. Verwendung von Ciclosporin in einer zur topischen Anwendung geeigneten Galenik zur Herstellung eines Arzneimittels zur Behandlung von Hautkrankheiten und/oder zur Behandlung von Krankheiten der Mundschleimhaut und/oder der Schleimhäute des Genitalbereichs, wobei das Ciclosporin in einer Öl-in-Wasser-Nanoemulsion vorliegt, wobei die Nanoemulsion einen Gehalt an Triacylglyceriden, vorzugsweise mittelkettigen Triacylglyceriden, aufweist, wobei als Emulgator lediglich Lecithin vorhanden ist und wobei es einen Gehalt an zumindest einem viskositätserhöhenden Zusatz aufweist.

2. Verwendung nach Anspruch 1 zur Herstellung eines Arzneimittels zur Behandlung von Lichen ruber.

3. Verwendung nach Anspruch 1 zur Herstellung eines Arzneimittels zur Behandlung von Neurodermitis.

4. Verwendung nach Anspruch 3 zur Herstellung eines Arzneimittels zur Behandlung von Neurodermitis im Bereich des Auges.

5. Verwendung von Ciclosporin in einer zur topischen Anwendung geeigneten Galenik zur Herstellung eines Arzneimittels zur Behandlung von Allergien, wobei das Ciclosporin in einer Öl-in-Wasser-Nanoemulsion vorliegt, wobei die Nanoemulsion einen Gehalt an Triacylglyceriden, vorzugsweise mittelkettigen Triacylglyceriden, aufweist, wobei als Emulgator lediglich Lecithin vorhanden ist und wobei es einen Gehalt an zumindest einem viskositätserhöhenden Zusatz aufweist.

6. Verwendung nach Anspruch 5 zur Herstellung eines Arzneimittels zur Behandlung von Allergien im Bereich des Auges.

7. Verwendung nach einem der vorhergehenden Ansprüche, **dadurch gekennzeichnet, dass** der Gehalt an Lecithin im Bereich von 0,1 bis 20 Gew.-%, vorzugsweise im Bereich von 1 bis 10 Gew.-% liegt.

8. Verwendung nach einem der vorhergehenden Ansprüche, **dadurch gekennzeichnet, dass** der Gehalt an Triacylglyceriden im Bereich von 10 bis 40 Gew.-%, insbesondere im Bereich von 20 bis 30 Gew.-% liegt.

9. Verwendung nach einem der vorhergehenden Ansprüche, **dadurch gekennzeichnet, dass** der Gesamtgehalt an Lipiden im Bereich von 1 bis 50 Gew.-%, vorzugsweise im Bereich von 20 bis 30 Gew.-% liegt.

10. Verwendung nach einem der vorhergehenden Ansprüche, **dadurch gekennzeichnet, dass** der Gehalt an Ciclosporin im Bereich von 0,1 bis 10 Gew.-%, vorzugsweise im Bereich von 1 bis 3 Gew.-% liegt.

11. Verwendung nach einem der vorhergehenden Ansprüche, **dadurch gekennzeichnet, dass** das Arzneimittel 2 Gew.-% Ciclosporin, 5 Gew.-% Lecithin, 23 Gew.-% mittelkettige Triacylglyceride und 70 Gew.-% physiologische Kochsalzlösung sowie gegebenenfalls ein physiologisch verträgliches Konservierungsmittel aufweist.

12. Verwendung nach einem der vorhergehenden Ansprüche, **dadurch gekennzeichnet, dass** die NanoemulsionTröpfchengrößen von kleiner als 500 nm aufweist.

13. Verwendung nach einem der vorhergehenden Ansprüche, **dadurch gekennzeichnet, dass** das Arzneimittel mit physiologisch verträglichen Trägerstoffen zum Auftragen auf die Haut vermischt vorliegt.

## Claims

1. Use of ciclosporin in a galenical preparation suitable for topical application for manufacturing a pharmaceutical for treating skin diseases and/or for treating diseases of the oral mucosa and/or the mucous membranes of the genital region, whereby the ciclosporin is present in an oil-in-water nanoemulsion, whereby the nanoemulsion has a triacylglyceride content, preferably medium-chain triacylglycerides, whereby as emulsifier only lecithin is present and whereby it contains at least one viscosity-increasing additive.

2. Use according to claim 1 for manufacturing a pharmaceutical for treating lichen ruber.

3. Use according to claim 1 for manufacturing a pharmaceutical for treating neurodermatitis.

4. Use according to claim 3 for manufacturing a pharmaceutical for treating neurodermatitis in the region of the eye.

5. Use of ciclosporin in a galenical preparation suitable for topical application for manufacturing a pharmaceutical for treating allergies, whereby the ciclosporin is present in an oil-in-water nanoemulsion, whereby the nanoemulsion has a triacylglyceride content, preferably medium-chain triacylglycerides, whereby as emulsifier only lecithin is present and whereby it contains at least one viscosity-increasing additive.

6. Use according to claim 5 for manufacturing a pharmaceutical for treating allergies in the region of the eye.

7. Use according to one of the previous claims, **characterised in that** the lecithin content lies in the range of 0.1% by weight to 20% by weight, preferably in the range of 1% by weight to 10% by weight.

8. Use according to one of the previous claims, **characterised in that** the triacylglyceride content lies in the range of 10% by weight to 40% by weight, preferably in the range of 20% by weight to 30% by weight.

9. Use according to one of the previous claims, **characterised in that** the total lipid content lies in the range of 1% by weight to 50 % by weight, preferably in the range of 20% by weight to 30% by weight.

10. Use according to one of the previous claims, **characterised in that** the ciclosporin content lies in the range of 0.1% by weight to 10 % by weight, preferably in the range of 1% by weight to 3% by weight.

11. Use according to one of the previous claims, **characterised in that** the pharmaceutical comprises 2% by weight of ciclosporin, 5% by weight of lecithin, 23% by weight of medium-chain triacylglycerides and 70% by weight of physiological normal saline solution and possibly a physiologically tolerated preservative.

12. Use according to one of the previous claims, **characterised in that** the nanoemulsion has droplet sizes of less than 500 nm.

13. Use according to one of the previous claims, **characterised in that** the pharmaceutical exists in a form mixed with physiologically tolerated carriers for application on the skin.

## Revendications

1. Utilisation de ciclosporine dans une forme galénique appropriée pour l'utilisation topique, pour la production d'un médicament pour le traitement des affections cutanées et/ou pour le traitement d'affections de la muqueuse buccale et/ou des muqueuses de la région génitale, la ciclosporine étant présente dans une nano-émulsion huile dans eau, la nano-émulsion présentant une teneur en triacylglycérides, de préférence des triacylglycérides à chaîne moyenne, de la lécithine étant présente uniquement comme émulsifiant et présentant une teneur en au moins un additif augmentant la viscosité.

2. Utilisation selon la revendication 1 pour la production d'un médicament pour le traitement du lichen ruber.

3. Utilisation selon la revendication 1 pour la production d'un médicament pour le traitement des névrodermites.

4. Utilisation selon la revendication 3 pour la production d'un médicament pour le traitement des névrodermites dans la région de l'oeil.

5. Utilisation de ciclosporine dans une forme galénique appropriée pour l'utilisation topique, pour la production d'un médicament pour le traitement des allergies, la ciclosporine étant présente dans une nano-émulsion huile dans eau, la nano-émulsion présentant une teneur en triacylglycérides, de préférence des triacylglycérides à chaîne moyenne, de la lécithine étant présente uniquement comme émulsifiant et présentant une teneur en au moins un additif augmentant la viscosité.

6. Utilisation selon la revendication 5 pour la production d'un médicament pour le traitement des allergies dans la région de l'oeil.

7. Utilisation selon l'une quelconque des revendications précédentes, **caractérisée en ce que** la teneur en lécithine se situe dans une plage de 0,1 à 20 % en poids, de préférence dans une plage de 1 à 10 % en poids.

8. Utilisation selon l'une quelconque des revendications précédentes, **caractérisée en ce que** la teneur en triacylglycérides se situe dans une plage de 10 à 40 % en poids, en particulier dans une plage de 20 à 30 % en poids.

9. Utilisation selon l'une quelconque des revendications précédentes, **caractérisée en ce que** la teneur totale en lipides se situe dans une plage de 1 à 50 % en poids, en particulier dans une plage de 20 à 30 % en poids.

10. Utilisation selon l'une quelconque des revendications précédentes, **caractérisée en ce que** la teneur en ciclosporine se situe dans une plage de 0,1 à 20 % en poids, en particulier dans une plage de 1 à 3 % en poids.

11. Utilisation selon l'une quelconque des revendications précédentes, **caractérisée en ce que** le médicament présente 2 % en poids de ciclosporine, 5 % en poids de lécithine, 23 % en poids de triacylglycérides et 70 % en poids de solution de sel physiologique ainsi que, éventuellement, un agent conservateur physiologiquement tolérable.

12. Utilisation selon l'une quelconque des revendications précédentes, **caractérisée en ce que** la nano-émulsion présente des gouttelettes inférieures à 500 nm.

13. Utilisation selon l'une quelconque des revendications précédentes, **caractérisée en ce que** le médicament se présente mélangé à des substances porteuses physiologiquement tolérables pour application sur la peau.
